Europäisches Patentamt

European Patent Office

Office européen des brevets

(19)

(11) Publication number: **0 155 476**
**A1**

(12) **EUROPEAN PATENT APPLICATION**

(21) Application number: **85101009.0**

(22) Date of filing: **31.01.85**

(51) Int. Cl.⁴: **C 12 N 15/00**
**C 12 N 7/00, C 12 N 5/00**
**//C12R1:91**

(30) Priority: **31.01.84 US 575453**

(43) Date of publication of application:
**25.09.85 Bulletin 85/39**

(84) Designated Contracting States:
**AT BE CH DE FR GB IT LI LU NL SE**

(71) Applicant: **IDAHO RESEARCH FOUNDATION**

**Moscow Idaho(US)**

(72) Inventor: **Miller, Lois K.**
**1187 Foot Hill Road**
**Moscow Idaho(US)**

(74) Representative: **Dipl.-Ing. Schwabe, Dr. Dr. Sandmair,**
**Dr. Marx**
**Stuntzstrasse 16**
**D-8000 München 80(DE)**

(54) **Production of polypeptides in insect cells.**

(57) A novel expression vector is employed in the expression of exogenous genes in insect cells utilizing recombinant DNA techniques. Exogenous genes under the control of strong promoters are inserted into viral chromosomes through the use of novel plasmids to form a viral vector which, in conjunction with the insect host is capable of expressing large segments of eukaryotic, prokaryotic or viral DNA in an eukaryotic environment.

EP 0 155 476 A1

Croydon Printing Company Ltd

SCHWABE · SANDMAIR · MARX

PATENTANWÄLTE

STUNTZSTRASSE 16 · 8000 MÜNCHEN 80

0155476

Anwaltsakte 50 512

## Production of Polypeptides in Insect cells

The present invention relates to methods and products
thereof for expressing an exogenous gene in insect cells,
and more particularly to the insertion of an exogenous
gene which codes for a desired gene product into the viral
chromosome of an insect virus such that it is under the
control of a strong gene promoter as intergrated therein,
introducing the viral vector into insect cells, and
expressing the desired gene product.

Interest in microbial insecticides has arisen as a result
of the problems associated with the use of chemical pesticides. Chemical pesticides generally affect beneficial as
well as non-beneficial species, and insects tend to
acquire resistance to such chemicals so that new insect
populations rapidly develop that are resistant to the
chemical pesticides. Furthermore, chemical residues pose
environmental hazards and possible health concerns. Thus,
microorganisms that are pathogenic to insects (entomopathogens) present an opportunity for an alternative means
of pest control and can play a role in integrated pest
management and reduce dependence on chemical pesticides.

Naturally occurring microorganisms or microbial by-products
have been identified as potentially useful insecticidal
agents. A number of entomopathogens have relatively
narrow host ranges, which makes it possible to reduce
specific pest populations while natural predators and beneficial insects are preserved or given the opportunity to
become reestablished. Entomopathogens which are useful for
insect control include certain bacteria, viruses and

-2-

fungi.

Viruses that cause natural epizootic diseases within insect populations have been commercially developed as pesticides. One such family of viruses that has been extensively studied is the Baculoviridae. Baculoviruses possess large (about 100 to 200 kilobases), double-stranded, circular, covalently closed DNA genomes that are packaged in enveloped, rod-shaped capsids approximately 40 to 140 by 250 to 400 nanometers. The term "baculovirus" is derived from the rod-shaped nucleocapsid structure which is characteristic of this family. A nucleocapsid is a unit of viral structure, consisting of a capsid (protein coat) with an enclosed nucleic acid.

The baculoviruses include the subgroups of nuclear polyhedrosis viruses (NPV) and granulosis viruses (GV). The virus particles of NPV and GV are occluded in proteinaceous crystals. In occluded forms of baculoviruses, the virions (enveloped nucleocapsids) are embedded in a crystalline protein matrix. This structure, also referred to as a polyhedral inclusion body, is the form found extracellularly in nature and is responsible for spreading the infection between organisms. The subgroup NPV contains many virions embedded in a single, large (up to 15 micrometers) polyhedral crystal, whereas the subgroup GV contains a single virion embedded in a small crystal. The crystalline protein matrix in both forms is primarily composed of a single 25,000 to 33,000 dalton polypeptide.

The NPV and GV baculovirus subgroups have been investigated for use as entomopathogens. The advantages of using viruses from the family Baculoviridae include: (1) they are known to cause lethal infections only in invertebrates; (2) they have a relatively narrow host range; (3) they produce sufficient progeny virus per insect to allow commercial production; and (4) the virus particles of NPV and GV are occluded in proteinaceous crystals which renders the viruses more stable in the environment, increases the shelf life as commercially prepared microbial pesticides, and facilitates combination with other pesticide formulations.

When used as pesticides, occluded viruses are usually sprayed on foliage. Insects that consume the contaminated foliage acquire the virus-induced disease. The ingested virus passes through the foregut of the insect to the midgut where the alkaline pH solubilizes the crystal. The virions are released from the matrix and begin the infection of the midgut columnar cells by fusion with microvillar membrane. Upon the death of the insect and the disintegration of the integument, the occluded viruses are released into the surrounding environments, and, if consumed by susceptible hosts, spread the infection.

The most extensively studied baculovirus is the Autographa californica nuclear polyhedrosis virus (AcNPV) which has a relatively broad host range. Viruses of this type replicate into two mature forms that are referred to as the non-occluded virus (NOV) form and the occluded virus (OV) or polyhedral inclusion body (PIB) form.

In both the NOV and OV forms, the nucleocapsids are enveloped by a membrane, but the precise nature of the envelopes is different. The nucleocapsids of the NOV acquire an envelope by budding through cellular membranes, generally the plasma membrane, whereas the nucleocapsids of the OV subgroup acquire a membrane envelope within the nucleus. The AcNPV nucleocapsids are usually enclosed within a single envelope when envelopement occurs in the

0155476

nucleus. Enveloped nucleocapsids within the cell nucleus may
be embedded in a crystalline protein matrix. The resulting
protein crystals, containing many enveloped nucleocapsids,
are known as occlusion bodies, polyhedral inclusion bodies
(PIBs), or polyhedra. The occlusion bodies of AcNPV are
approximately one to four micrometers in diameter, allowing
visualization with light microscopy. Each virion consists
of one or more nucleocapsids enveloped in a single membrane.
The morphological feature, whereby there may be more than
one nucleocapsid per envelope, is characteristic of a type
of NPV known as multiple-embedded virus to distinguish it
from NPVs in which a single nucleocapsid is individually
enveloped. From a genetic engineering standpoint it is
important that the presence of multiple nucleocapsids per
envelope is characteristic of only the occluded form of an
NPV and not the non-occluded form, since it is possible to
clone and genetically manipulate the virus more easily
using the non-occluded form.

The AcNPV DNA genome (128 kilobases) has been mapped with
respect to restriction sites for various restriction endonucleases, and is primarily composed of unique nucleotide
sequences. See L.K. Miller et al., Science Vol. 219:715 -
721 (11 Feb. 1983).

The OV form of AcNPV is not infestious in cell culture
unless virions are released from the occlusion body by alkaline disruption. Thus, OVs formed in cell culture are
non-infectious in cell-cultures. Rather, NOVs are responsible for the spread of infection from cell to cell.
As the infection becomes more extensive the virus replicates in the midgut cells and produces NOVs that bud
through the basement membrane into the hemolymph of the
insect. The non-occluded virions that are released into
the hemolymph are responsible for the systemic infection
in the insect, as well as for spreading the infection in cell cultures.

The synthesis of virus induced proteins in AcNPV infected cells is a temporally controlled process. There are four stages of viral-induced protein synthesis: (1) an early alpha phase approximately two to three hours after infection; (2) an intermediate beta phase (six to seven hours after infection) requiring functional protein and DNA synthesis; (3) a gamma phase (at ten hours) including synthesis of virion structural proteins; and (4) a late delta phase (at fifteen hours) associated with occlusion.

Infectious non-occluded virions of AcNPV are found in the cuture medium approximately ten hours after infection. The synthesis of polyhedrin, the major protein of the occlusion matrix, has been detected by twelve hours after infection and this preceeds the appearance of occluded virus at sixteen or eighteen hours after infection.

Temporal control of transcription is probably responsible for the temporal control of AcNPV protein synthesis. The transcription of the polyhedrin gene is first observed at twelve hours after infection, and the 1.2 kilobase transcript progressively increases in quantity up to twenty-four hours or more after infection. In cell culture, NOV release usually preceeds occlusion. In the case of AcNPV in nature, the infection is so pervasive that as much as 10% of the weight of a dead infected host organism may be due to occluded virus. The 10% weight relationship indicates truly massive production of the occluded form of the virus, and in conjunction therewith, massive production of polyhedrin protein.

From a genetic engineering standpoint, the genes controlling occlusion are not necessary for infection in cell culture, and the elimination or replacement of these genes provides an opportunity for substitution of passenger DNA into the regions controlling occlusion and this could provide a high level of expression of passenger genes at late times in the infection cycle. The crystalline polyhedrin protein constituting the matrix of the occlusion boyd of AcNPV is predominately a single protein of approximately 30,000 daltons. About 95% of the weight of an occlusion body consists of polyhedrin. Thus, polyhedrin is synthesized in extremely large quantities as evidenced by the high proportion of the virus in the body weight of the infected host. In addition, the increased level of synthesis of this single protein is illustrated by cells infected with wild type strain AcNPV which produces an average of over 60 occlusion bodies per cell, each occlusion body being approximately 4 micrometers in diameter. Thus the promoter for polyhedrin mRNA synthesis appears to be exceptionally strong.

A. california nuclear polyhedrosis virus has been proposed as a vector for recombinant DNA research and genetic engineering in invertebrates. See L.K. Miller, J. Virol 39:973, the disclosure of which is incorporated herein by reference. However, until the achievement of the present invention, it has not been possible to use that virus, or any other insect virus, as a vector.

Accordingly, it is an object of the present invention to provide a vector which is useful in expression of exogenous materials in insect cells.

Summary of the Invention

In accordance with the present invention, an expression vector is employed for the first time in expressing ex-

genous gene products by insect cells utilizing recombinant DNA techniques. As used in this application, the term "gene product" means the end product of a gene's expression and includes RNAs whose role may be structural or enzymatic in nature and the translational products of mRNAs, i.e. polypeptides, whether or not glycosylated, whose role may be structural or enzymatic in nature. This definition is also meant to include RNAs and polypeptides which play a regulatory role in gene expression. This invention provides novel means of employing expression vectors, e.g. plasmids or viral vectors, preferably an insect or entomopathogen virus expression vector, for expression of an exogenous gene product which is under the control of an effective promoter, preferably a promoter which promotes expression of a gene product in insect cells. More specifically, in a preferred embodiment, the present invention comprises cloning the region of an NPV (such as AcNPV) surrounding the polyhedrin gene, providing a plasmid which includes the polyhedrin gene region, inserting an appropriate passenger gene into the polyhedrin gene or replacing at least a portion of the polyhedrin gene with an appropriate passenger DNA while in plasmid form, co-transfecting the resulting passenger plasmid DNA with wild type AcNPV DNA into suitable host cells and use of the transformed host to produce the exogenous gene product. Suitably transformed host cells can be identified, e.g. by selecting for a virus having a phenotype where an allelic replacement of the polyhedrin gene with passenger DNA has resulted in a virus defective in occlusion body formation.

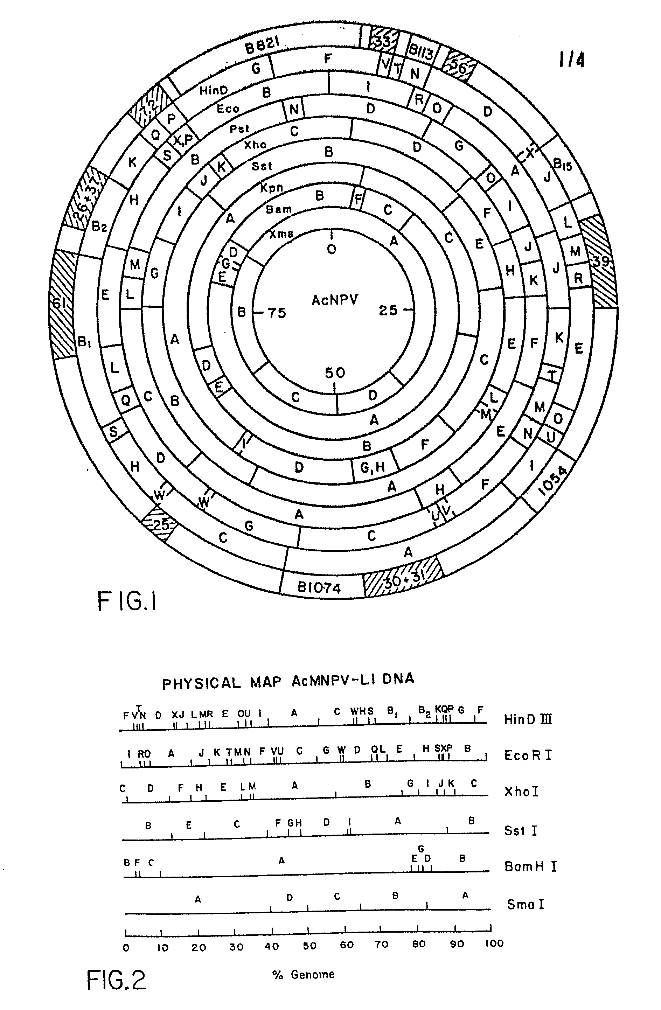

Another promoter for use in connection with the present invention is the promoter controlling the synthesis by A. californica of a 7,200 dalton protein, termed simply the "7.2K protein". See Adang and Miller, J. Virol 44:782 - 793, 1982. Like the polyhedrin protein, this protein is expressed late in infection during the occlusion phase of virus replication. While the exact function of this protein in occlusion is not yet known, the gene which produces the protein has been located and identified, and thus is available for use. See Figure 1. As used in this application the term "7.2K promoter" means the promoter which controls the gene which is responsible for expression of that 7.2 Kd protein. Other promoters, from AcNPV or elsewhere, can also be used to produce exogenous gene products in insects, including other promoters from Baculoviridae, as well as other viruses, bacteria, yeasts, other fungal, insect and even mammalian promoters. Those promoters which are functional in insect cells may be identified, e.g. by replacement of the AcNPV promoter pMC 874 described infra, transfected into insect cells of interest, and beta-galactosidase production monitered, as disclosed herein.

Thus, valuable host/vector systems are provided which can be employed for propagating and expressing large segments of eukaryotic, prokaryotic or viral passenger DNA in a eukaryotic environment. Thus, the present invention permits exogenous passenger DNAs, including those with gene products causing cell death, to be propagated and expressed at a high level in an invertebrate environment.

Brief Description of the Drawings

This invention can be more clearly understood by referring to the accompanying drawings wherein:

Fig. 1 is the physical map and gene organization of the baculovirus AcNPV.

Fig. 2 shows a linear representation of the restriction endonuclease recognition sites in the circular DNA of the L-1 varient of AcNPV.

Fig. 3 is a physical map of the pEXX942 plasmid DNA.

FiG. 4 is a physical map of the pGP-B6874/SAL plasmid DNA.

Fig. 5 is a physical map of the pGP-B6874 plasmid DNA.

Fig. 6 shows the PstI and BamHI patterns of AcNPV L1GP-Gal3.

Fig. 7 shows the schematic construction of pGP-B6874/SAL utilized as a transplacement vector wherein the exogenous passenger DNA of the transplacement vector expresses chloramphenicol acetyl transferase and is under the control of the exogenous promoter RSV-LTR, obtained from Rous sarcoma virus, incorporated therein.

Detailed Description of the Invention

In one embodiment of this invention, a method of using Autographa california nuclear polyhedrosis virus (AcNPV) as an expression vector has been developed wherein a segment of AcNPV DNA encompassing the polyhedrin gene is cloned in E. coli using a plasmid vector. As mentioned above, AcNPV has been extensively studied. Samples of AcNPV may be obtained from numerous sources, including the Yale Arbovirus Research Unit (YARU) located in New Haven, Conneticut. A restriction enzyme is used to open up the polyhedrin gene on the recombinant plasmid DNA to create a site for insertion of passenger DNA. The passenger DNA is selected and inserted into the cloned plasmid at the site opened by the restriction enzyme. The plasmid is then introduced, e.g. transfected, along with wild type AcNPV DNA into suitable host cells. Thereafter, either by gene conversion or a double recombination event , the DNA from the recombinant plasmid replaces its allelic counterpart, that is, the polyhedrin gene, on the full length wild type AcNPV chromosome. Hence, when RNA sythesis occurs in the virus, the messengerRNA read from the inserted passenger DNA serves to express a new gene product under its direction. Specifi~
viruses containing the passenger DNA may be selected by
any appropriate technique. The selected virus may then
be propagated to exploit the ability of this passenger
DNA to direct the sythesis of the desired gene product.

Fig. 1 represents the physical map and gene organization
of the baculovirus AcNPV. The physical map of the restriction endonuclease sites of the 128 kilobase circular, double-stranded DNA genome is presented in the
inner eight concentric rings. The outer concentric circle
presents information on the gene organization of AcNPV.
The stippled areas refer to the position of some of the
temperature-sensitive mutations, and the hatched areas
refer to regions encoding various structural and non-
structural proteins of AcNPV with the size of the proteins given in the number of kilodaltons. Note that the
approximately 33,000 dalton gene for polyhedrin protein
is located in the outer ring at the zero to approximately
the 10% position, and the gene for an approximately
7,200 dalton protein ("7.2"), which is also expressed
in late stages, is located in the outer ring at about
the 85-90% position.

Fig. 2 is a linear representation of the restriction
endonuclease recognition sites in the L-I variant of
AcNPV. The L-1 variant has an additional Hind III site
within Hind III-B and the two fragments are referred to
as B1 and B2 with the size of the total genome being
approximately 128 kilobases.

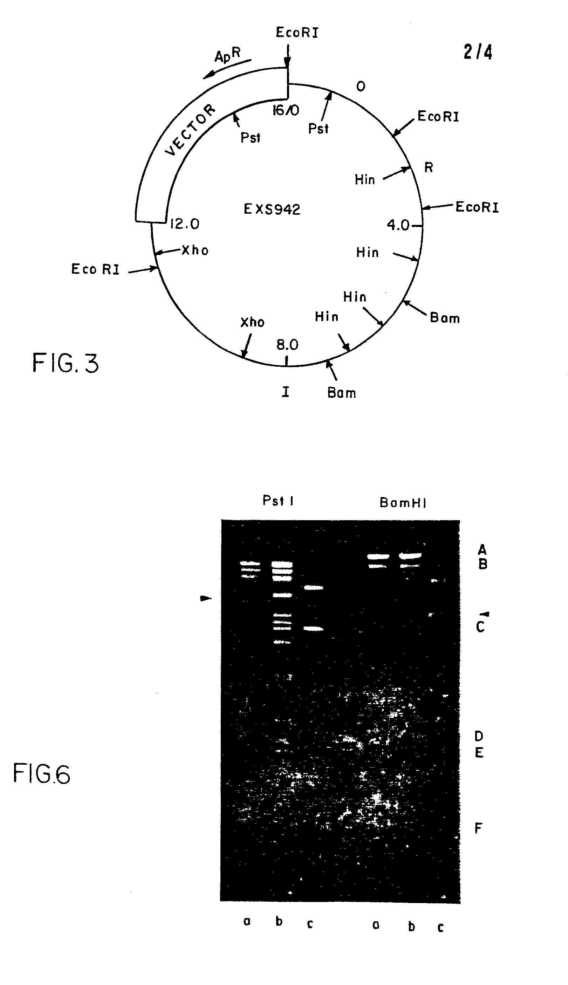

Fig. 3 represents a physical map of the pEXS942 plasmid
DNA which contains the 0.0 to 8.7 region of the AcNPV
L-1 DNA, including EcoRI-I, R and 0 fragments. The vector
segment was derived by EcoRI and SalI digestion of pBR325.

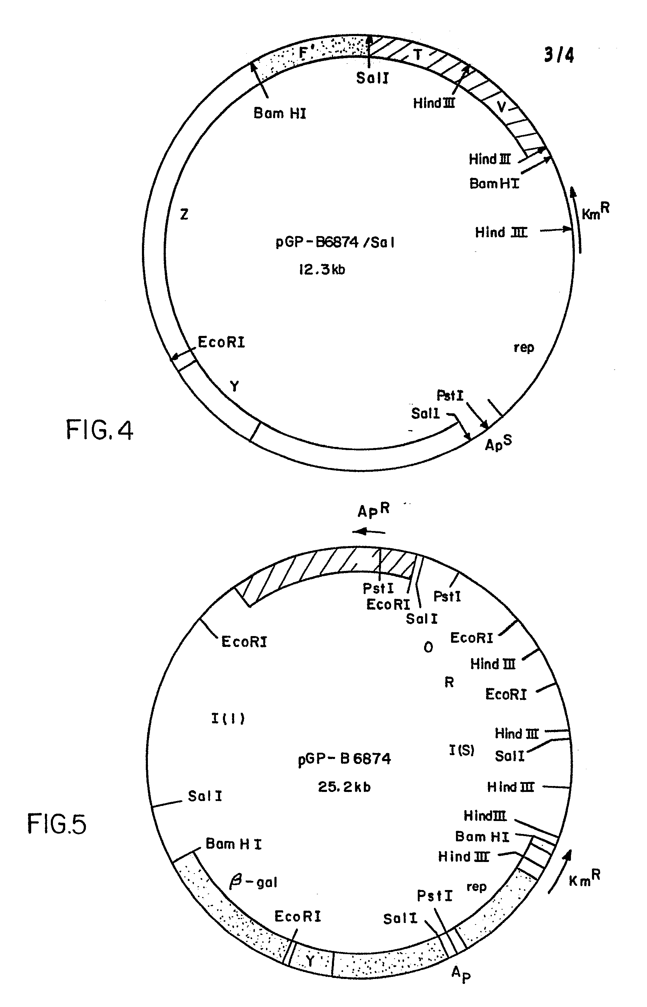

Fig. 4 is a physical map of the pGP-B6874/Sal plasmid
DNA having a total weight of 12.3 kilobases and containing a gene for kanamycin resistance.

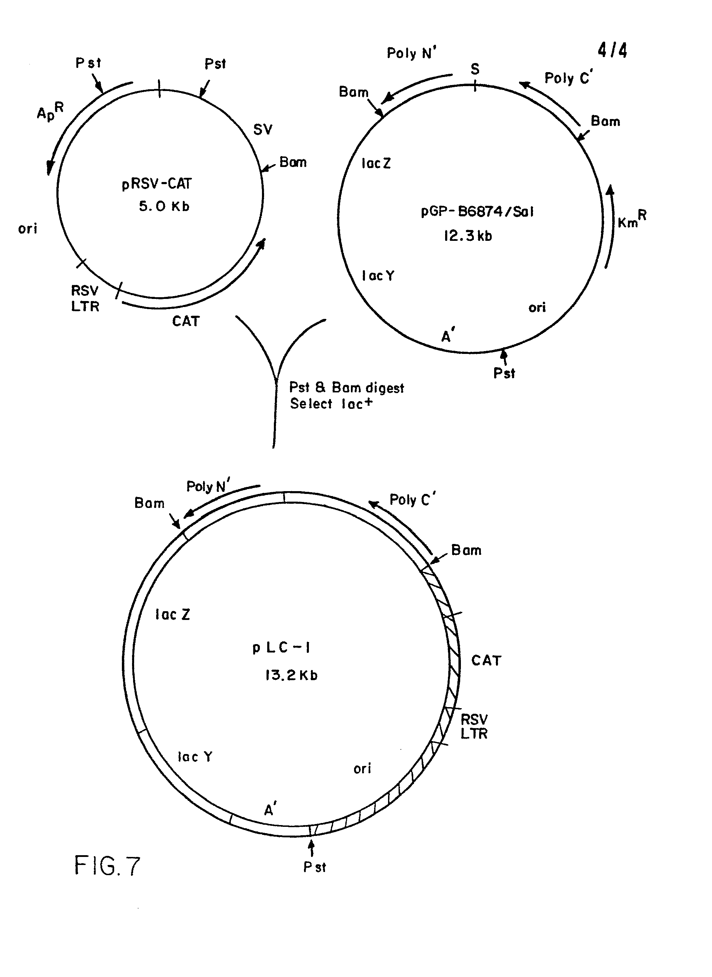

Fig. 5 is a physical map of the pGP-B6874 plasmid DNA
having a total weight of 25.2 kilobases and containing
a gene from E. coli which codes for the protein beta-
galactosidase.

It has been found that in accordance with one aspect
of the present invention, baculoviruses are useable as
vectors for propagating and expressing exogenous DNA in an
invertebrate eukaryotic environment. Baculoviruses are
highly advantageous as recombinant DNA vector systems
because they possess such features as: (1) a covalently-
closed, circular nuclear-replicating DNA genome, (2)
an extendable rod-shaped capsid which may increase in
size to accept an additional 20 kilobase or larger DNA
segment, (3) a group of genes that are involved in
occlusion yet are non-essential for infectious virus
production and thus may be deleted, and (4) one or more
strong promoters which are turned on after infectious
virus production and control the synthesis of proteins
involved in occlusion (e.g. polyhedrin and 7.2K protein)
which constitute 10% or more of the protein of infected
cells. AcNPV is employed in the method of this invention
by using a plasmid vector containing a passenger DNA
to replace the viral DNA genome that codes for occlusion
protein synthesis. Thus the passenger DNA, once it is
included in the viral DNA, has the advantage of the
strong polyhedrin and/or 7.2K protein promoter, which,
once turned on, will express the gene product encoded for
in the passenger DNA in desirable amounts. It has also
been found that, where desirable, the passenger DNA may alternatively be under the control of its own or other exogenous promoters as discussed herein below. Finally, viruses that have expressed the passenger DNA may be easily selected and propagated.

In a preferred embodiment, the first step in utilizing the polyhedrin promoter to express exogenous DNA is to clone a vector containing the AcNPV polyhedrin gene into an appropriate host. The location of the 3' terminal 62% of the 1.2 kilobase polyhedrin gene of AcNPV is located in the Hind III-V region of AcNPV as indicated in Figs. 1 and 2. Thus a plasmid for the replacement of the polyhedrin gene with a passenger DNA is begun by cloning into E. coli, using a plasmid vector a segment of AcNPV DNA from 0.0 to approximately 6.0 Kd. This will encompass at least the 3' terminal segment of the polyhedrin gene (which occurs at about 3.3 to 4.0 Kd), if not the entire gene. A restriction endonuclease that recognizes a site or sites whithin the polyhedrin gene region may then be used to open the recombinant plasmid DNA, creating a site for the insertion of passenger DNA. It is noted that the restriction endonuclease BamHI, has some useful sites in this region. Once the passenger DNA is inserted into the cloned plasmid the plasmid can be used in a "marker rescue" fashion. See L.K. Miller, Journal of Virology Vol. 39:973 (1981). Using this technique, full length AcNPV DNA is co-transfected with the fragment of AcNPV DNA containing the passenger DNA presented by the plasmid. Allelic replacement occurs either by gene conversion or a double recombination event resulting in the replacement of DNA in the full length viral DNA with DNA from the fragment presented by the plasmid. Thus, the natural polyhedrin gene is replaced with the engineered polyhedrin gene passenger DNA, and selection for passenger DNA-containing viruses may be performed by screening plaques for absence of occlusion bodies, e.g. using visual observation of virus plaques under the light microscope. An exact size replacement of any deletions of the polyhedrin gene is not necessary since the rod-shaped nucleocapsid of baculoviruses is extendable to greater lengths.

In many instances, it is preferable to have the polyeptide or other gene product which is expressed in accordance with this invention secreted outside the cell membrane of the insect cells in which it is expressed. This can be accomplished in known manners, e.g. by encoding en appropriate leader sequence which is expressed in conjunction with the polypeptide sought, and which enables that product to be secreted by the cell.

In order to insert a passenger DNA into AcNPV L-1, the 0.0 to 8.7 region of AcPV L-1 DNA in E. coli is cloned using pBR325 as a vector. The AcNPV L-1 is a wild type virus which may be used in this recombinant DNA construction and its physical map is shown in Fig. 2. See Miller, et al. Virology Vol. 126:376-380 (1983), the disclosure of which is incorporated herein by reference. The plasmid pBR325 (described by Bolivar in Gene Vol. 4, p. 121 (1978) is a derivative of plasmid pBR322 and carries unique EcoRI sites for selection of EcoRI generated recombinant DNA molecules. A recombinant plasmid may be constructed by digesting pBR325 with EcoRI and SalI restriction endonucleases which cleave within the chloramphenicol resistance and tetracycline resistance genes, respectively, leaving the ampicillin resistance gene and replication origin intact with one EcoRI cohesive end and one SalI cohesive end. AcNPV L-1 DNA may be digested simoultaneously with EcoRI and XhoI. Following ligation of the EcoRI, XhoI, or SalI digested viral/pBR325 fragmentDNAs, E.coli is transformed. $Ap^R$ $CM^S$ $TET^S$ colonies may be screened for plasmids containing BamF. During
screening procedures, pEXS942 has been found and, it is
believed, arose due to the co-cloning of an AcNPV Xho-EcoRI
fragment and an EcoRI partial digestion product containing
EcoRI-I, R and O. Since pEXS942 contains the region from
0.0 to 8.7, rather than 1.9 to 5.9, there is provided
a larger region of the AcNPV genome and more DNA on both
sides of the Hind III-V region (3.3 to 4.0). It is believed that this larger region facilitates the double
recombination (or gene conversion event) necessary for
marker rescue between plasmid DNA and AcNPV DNA in vivo.
Another advantage is the presence of the viral PstI site
at 8.0 which is useful in further constructions to insert
passenger DNA into the polyhedrin region. Finally, the
0.0 to 8.7 region totally encompasses the Hind III-N
region of AcNPV which includes tsB113 which is a
temperature sensitive mutant. Thus, tsB113 and pEXS942 can
be used together as additional selection for allelic
replacement.

To determine if the region of AcNPV L-1 encompassing
BamF could be deleted in its entirety, the pEXS942 DNA
was digested with BamHI and religated so as to delete
the BamF fragment. Among the plasmids obtained, a novel
construction was isolated and hereafter referred to as
pEXS942 B6. This plasmid is not deleted for Bam F, but
rather lacks the BamHI site at 4.5. Thus, pEXS942 B6 has
only a single BamHI site at 3.0 map units, since the
BamHI site was eliminated at 4.5. In this procedure, the
region of AcNPV surrounding the polyhedrin gene is cloned
in E. coli and from that a plasmid pEXS942 B6 may be
isolated. This plasmid includes the AcNPV region from
0.0 to 8.7 map units, thus encompassing at least the
polyhedrin 3' terminal 62% between 3.3 and 4.0. Thus,
the polyhedrin region in pEXS942 B6 or its derivatives can be replaced with an appropriate passenger DNA while
in plasmid form. The resulting pEXS942 B6 passenger
plasmid DNA is then co-transfected with the wild type
AcNPV genome. A double recombination or gene conversion
event between the recombinant plasmid DNA and wild type
AcNPV results in allelic replacement of the polyhedrin
gene of AcNPV with passenger DNA. Successful allelic
replacement of the polyhedrin gene with passenger DNA
results in viruses defective in occlusion body formation.
This phenotype may be selected with appropriate plaque
assays. In addition, a mutation in an AcNPV mutant,
tsB113, maps adjacent to the polyhedrin gene so that
successful allelic replacement within this region is
selectable by decreased temperature sensitivity. The
host/vector system may be employed for a variety of
purposes once an appropriate restriction site for
passenger DNA insertion has been identified.

The advantages of the AcNPV vector system include the
ability to package large segments of passenger DNA in
the rod-shaped viral capsid and the availability of
strong promoters such as the polyhedrin and 7.2K protein
promoters which are induced following the production of
infectious non-occluded virus. An additional advantage
of placing an exogenous passenger DNA in this position
is that sythesis for these promoters occur late in the
infection cycle, subsequent to the onset of NOV release,
and more than 24 hours prior to cytolysis. As a result,
infectious virus production is not impaired by any
active passenger gene product. Long periods of cell
viability have been achieved, which suggests continued
metabolic functioning during passenger protein synthesis .

The genome of AcNPV is a double stranded, circular,
covalently-closed DNA molecule of approximately 82,000,000 daltons. These features include a rod-shaped capsid that may accommodate 20 kilobases or more of passenger DNA and a sequential expression of viral genes resulting in the early production of infectious virus followed by a lengthy period of virus directed protein synthesis from a number of promoters.

In another embodiment of the present invention, the promoter controlling the synthesis of the 7.2K protein may also be utilized for passenger gene expression. The 7.2K protein gene is located between 88 and 91 map units in Figure 1. See Adang and Miller, J. of Virology 44:782 (1982), the disclosure of which is incorporated herein by reference. Synthesis of the mRNA encoding the 7.2K protein extends from Hind III-Q through Hind III-P. A plasmid containing most of the DNA encoding the 7.2K protein has been cloned (Adang and Miller, supra) and could be used for passenger gene insertion and allelic replacement within the 7.2K protein gene.

In other embodiments of the invention, it is possible to introduce passenger genes under the control of exogenous (non-baculovirus) promoters, such as promoters from other viruses, bacteria, fungae, insects, mammals, etc. In this approach, the passenger gene is attached to an exogenous promoter and together the exogenous promoter-gene combination is inserted into an appropriate "transplacement vector". The transplacement vector is designed so as to permit allelic replacement of an appropriate region of viral DNA. The plasmid pGP-B6874/Sal (Fig. 4) is one such transplacement vector. This plasmid has a single Pst I site in a non-essential region of the plasmid. An exogenous promoter gene can be inserted into this Pst I site. The resulting recombinant plasmid is co-transfected with wild type viral DNA and recombinants selected by an appropriate technique. In the case of pGP-B6874/Sal, the vector is designed so that a blue color is produced for recombinant viruses resulting from allelic replacement. One example of an exogenous promoter is the long terminal repeat promoter of Rous sarcoma virus, an avian sarcoma virus which is widely available. This promoter (RSV-LTR) has been inserted into AcNPV and shown to direct the synthesis in insect cells of an exogenous protein, such as the chloramphenicol acetyl transferase gene of E. coli. One advantage of the use of an exogenous promoter of this nature is that the promoter is activated at early as well as late times in the viral replication process. Early expression may be useful in some applications of genetic engineering of baculoviruses and/or insect cells, particularly with respect to the use of baculoviruses as biological pesticides. Where large scale production of polypeptides using insect cells is the goal, early producers would be advantageous, e.g. where the protein produced would not adversely affect cell viability or productivity.

The following examples are given to illustrate embodiments of the invention as it is presently preferred to practice it. It will be understood that these examples are illustrative, and the invention is not to be considered as restricted thereto except as indicated in the appended claims.

## Example 1

AcNPV can be successfully employed as a recombinant DNA vector by stably propagating a 9.2 Kb passenger DNA inserted into the polyhedrin gene. In addition the polyhedrin promoter drives the high level expression of a passenger gene, such as E.coli beta-

0155476

galactosidase.

The plasmid pEXS942 B6 contains the 0.0 to 8.7 map unit region of AcNPV but lacks the BamHIsite at the BamF/C junction, and thus contains only a single BamHI site near or within the polyhedrin gene.

The pMC874 plasmid possesses a single BamHI site at the eight codon of the E. coli beta-galactosidase gene in the same reading frame as the BamHI site of the polyhedrin gene of beta-galactosidase. See Casadaban et al., J. Bact. 143:971-80 (1980). In addition to the beta-galactosidase gene, the 9.2 Kb pMC874 plasmid contains the lac Y gene, part of the lac A gene, a portion of the ampicillin resistance gene, a Col El replication origin, and a gene for kanamycin resistance. A fusion of BamHI digested pMC874 and plasmid pEXS942 B6 DNAs by in vitro ligation results in the formation of a 25.2 Kb plasmid, pGP-B6874 shown in Fig. 5, and hereafter called the fusion plasmid.

The 25.2 Kb plasmid pGP-B6874 was constructed by digesting pEXS942 B6 and pMC874 with BamHI followed by ligation at high DNA concentrations to favor bimolecular fusions. See Maniatis et al., Molecular Cloning, Cold Spring Harbor Laboratory (1982), the disclosure of which is incorporated herein by reference. Following transformation of E. coli HB 101 with the ligation products, the plasmid fusions were selected by their resistance to ampicillin and kanamycin. The ampicillin resistance gene is supplied by pEXS942 B6 and the kanamycin resistance gene is supplied from the pMC874. Blue colonies on plates containing ampicillin, kanamycin and X-gal (a colorimetric indicator for beta-galactosidase enzymatic activity) were selected.

For plaque assays involving blue color production, a procedure similar to plaque assay procedures involving
neutral red staining of lepidopteran cell monolayers
was adapted. See Lee and Miller, J. Virol. 27:754-67
(1978). The procedure used for blue color production was
similar except that 120 micrograms per milliliter of
X-gal was included in the agarose overlay media. Instead
of neutral red staining, the plaques are visualized by
their blue color which is enhanced by warming the plates
at 37 degrees C. for 4 - 6 hours, or allowing the color
to develop overnight at room temperature. Alternatively
for quick visualization, the tissue culture dishes are
frozen in liquid nitrogen for five to ten seconds, thawed
at room temperature, and the color allowed to develop
for two to three hours. Infectious viruses were picked
from the plaques, and replaqued directly or grown into
a virus stock.

The colonies which contained the fusion plasmid pGP-B
6874 (Fig. 5) with the beta-galactosidase gene fused to
the polyhedrin N-terminus were demonstrated by restriction endonuclease digestion with Sal I, Eco RI and Hind
III enzymes. These enzyme analyses distinguish the two
possible orientations of the fused plasmids.

To transfer the polyhedrin/pMC874 fusion sequences (now
represented by the fusion plasmid pGP-B6874) into the
AcNPV DNA genome, allelic replacement or"transplacement"
technique is employed. See L.K. Miller, J. Virol.
39:973-76 (1981), the disclosure of which is incorporated
by reference. Monolayers of S. frugiperda cells were co-
transfected with intact wild type AcNPV and a 100-fold
molar excess of pGP-B6874 DNA using a calcium phosphate
co-precipation technique. See Potter and Miller,
J. Invert. Path. 36:431-2 (1980), the disclosure of which is incorporated herein by reference. Viruses expressing the beta-galactosidase gene were detected as blue colored plaques in the presence of X-gal.

Plasmid DNAs were prepared by a rapid clear lysate procedure. See Holmes and Quigley, Anal. Biochem, 114:193 (1981). AcNPV DNA was prepared from extracellular virus which was partially purified from infected culture media by centrifugation through a 20% sucrose solution at 90,000 x g for 90 minutes at 5°C. The pellet was resuspended in 200 microliters of 10 mM Tris-HCl pH 7.6, 10 mM EDTA and 0.25% SDS. Proteinase K was added to a final concentration of 0.5 mg/ml and the mixture was incubated for 30 minutes at 30°C and then phenol extracted twice before the DNA was precipitated.

The wild type virus used in the recombinant DNA construction was the L-1 variant of AcNPV referred to above and isolated and described in Lee and Miller, J. Virol. 27:754-767 (1978), the disclosure of which is incorporated herein by reference. A continuous cell line of a lepidopteran noctuid Trichoplusia ni, TN-368, was propagated in TC-100 media (Microbiological Associates) supplemented with 0.26% Tryptose broth, 10% fetal bovine serum, 2 mM L-glutamine and an antibiotic-antimycotic preparation (Gibco).

Comparison of neutral red-stained monolayers and X-gal-stained monolayers indicated that approximately one in a thousand of the plaques resulting from co-transfection of AcNPV and pGP-B6874 expressed beta-galactosidase. Several blue plaques were chosen and directly replaqued on fresh S. frugiperda cell monolayers.

Transfections were performed by the method of Potter and Miller, supra, using the calcium phosphate precipitation method without the glycerol boost step. Following the 20 minute incubation of the DNA precipitate, the S. frugiperda cells were overlaid with liquid medium for two to three hours and this overlay was then replaced with either fresh liquid medium or agarose-containing medium for plaque development. For transplacement experiments, a molecular ratio of 100 plasmid DNAs per viral DNA was routinely used. The DNAs to be used in the transfection procedures were stored in water rather than a Tris-EDTA solution.

Blue plaques, free of occlusion bodies, were picked, developed into virus stocks, and characterized by restriction endonuclease analysis. The Pst I and BamHI patterns of one of these viruses, AcNPV LlGP-gal3, is presented in Fig. 6. Pst-D is missing in LlGP-gal3, and two new fragments are present. One new fragment is found between Pst-C and D and the other is found below Pst-F. The latter fragment co-migrates with a Pst I fragment of pGP-B6874 from the ApS region of pMC874 to the Eco RI-0 region of AcNPV. The new Pst fragment between Pst C and Pst D corresponds to the Pst I-0/N junction in Eco RI-B through Eco RI-I of AcNPV (1) and the E. coli beta -gal, lac Y and lac A region. The BamHI pattern also confirms that the entire pMC874 is properly inserted into AcNPV. No BamHI fragments from the wild type strain AcNPV are present in LlGP-gal3 but a new 9.2 kilobase fragment, co-migrating with pMC874, is found. BamF has also been observed in the LlGP-gal3 virus, indicating that homologous recombination between AcNPV and pGP-B6874 occurred near the pMC874 and the Eco RI-I (s) junction. Thus, the virus AcNPV LlGp-gal3 expresses beta-galactosidase activity, indicating that the beta-galactosidase gene originating from pMC874 has been found to recombine into this virus.

Regulation of Expression of Beta-Galactosidase Activity

To determine if the expression of the beta-galactosidase gene was under the same temporal regulation as polyhedrin gene expression, monolayers of S. frugiperda were infected with wild type AcNPV or AcNPV L1GP-gal3 at a multiplicity of infection of 20 plaque forming units (PFU) per cell. At various times post infection, the infected cells were assayed for beta-galactosidase activity and total protein content. Beta-galactosidase activity first appears between 18 and 24 hours and then dramatically increases through 48 hours after infection.

Levels of beta-galactosidase were determined using the ortho-nitrophenylgalactoside (ONPG) assay described by Miller in Experiments in Molecular Genetics, Cold Spring Harbor Laboratory, New York (1972). Insect cells tested were infected with a virus at a multiplicity of infection of 20 PFU/cell. The virus was allowed to adsorb for 1 hour at room temperature with gentle rocking. The innoculum was removed and replaced with appropriate media and incubation was carried out at 27 degrees centrigrade for the time indicated. The cells were washed with a phosphate-buffered saline solution (Lee and Miller, supra) and cells were scraped from the plate and pelleted at 200 x g for five minutes. The supernatant was discarded and the cell pellet frozen in liquid nitrogen and stored at minus 70°C until assayed. The cells were resuspended in distilled water and then diluted appropriately (from 1:1 to 1:1000) in Z buffer so a faint yellow color developed between 15 minutes and 1 hour after ONPG addition. The cells were disrupted using chloroform and 0.1% SDS as recommended by Miller, 1972. Specific activity was defined as n moles ONPG cleaved/min/mg protein.

The observed specific activity at 48 hours post infection is 8,000 n moles of ONPG cleaved per min/per mg. protein, which represents an approximate 900-fold increase in beta-galactosidase activity. A specific activity of 8,000 means that at least 3% of the total cellular protein is beta-galactosidase since pure beta-galactosidase has a specific activity of $3 \times 10^5$ units per mg. At 72 hours post-infection, the specific activity drops probably due to the onset of cytolysis.

Regulation of polyhedrin/beta-galactosidase gene expression was also analyzed by pulse-labelling infected cells with $35_S$ methionine at various times post infection. The $35_S$ labelled proteins are analyzed by SDS-polyacrylamide gel electrophoresis. Coomassie blue staining of the proteins shows that early in the infection there is no significant difference between the wild type or the L1GP-gal3 profiles. From 18 to 72 hours post infection however, the 33,000 dalton polyhedrin protein is synthesized in wild type infected cells but not in L1GP-gal3 infected cells.

L1GP-gal3 profiles show a new protein of approximately 120,000 daltons as well as several smaller proteins in the 95,000 dalton range at about 18 hours post infection that are not found in wild type infected cells. The largest protein is the appropriate size for a polyhedrin/ beta-galactosidase fusion polypeptide. The intensity of the Coomassie blue stain in the 120,000 dalton region corroborates the specific activity estimate that active beta-galactosidase constitutes at least 3% of the total cellular protein at 48 hours post infection. Coomassie blue stain also reflects the reduction in the amount of the polyhedrin/beta-galactosidase fusion protein at 72 hours post infection.

The procedure of Miller, et al, 1983, supra is used for analysis of virus-induced proteins in infected S. frugiperda cells except that $1 \times 10^6$ cells per 35 milimeter plate were used and the cells were washed with media after pelleting at low speed.

Immune Precipitation with Polyhedrin Antibody

To further explore the nature of the polypeptides produced at 48 hours in wild type and the L1GP-gal3-infected cells, the 35 S-labeled proteins at 48 hours post infection were precipitated with antiserum raised to purified polyhedrin. Immune precipitation using polyhedrin antiserum was performed by a slight modification of the procedure of Kessler, Immunology 117:1482-1490 (1976). Forty microliters of 35 S-labeled protein from 48 hours post infection were incubated with 40 microliters of polyhedrin antiserum for 1 hour at room temperature and then 160 microliters of a 2% suspension of the Staphylococcus aureus (Sigma) in precipitation buffer (0.15 M NaCl, 5 mM EDTA, 50 mM Tris-Hcl, 0.02% NaN3, 0.05% NP40, and 0.1% BSA) was added. The mixture was incubated at room temperature for 20 minutes and the resulting precipitate was collected by centrifugation at 15,000 x g for 1 minute. The supernatant was discarded and the pellet was washed with 0.5 milliliters of precipitation buffer. The pellet was then resuspended in solubilization buffer (1% SDS, 0.5% beta-mercaptoethanol and 0.5 M urea) with vortexing and heated for 5 minutes at 100°C. The debris was pelleted at 15,000 x g for five minutes and the supernatant was analyzed by SDS-polyacrylamide gel electrophoresis.

The polyhedrin antiserum precipitated the 33,000 dalton polyhedrin protein from wild type infected cells as well as several polypeptides in the 20,000 to 25,000 dalton region. Polypeptides of this size were not precipitated by polyhedrin antiserum in the L1GP-gal3-infected cells. Instead, the 120,000 protein was the predominant precipitant as well as the series of polypeptides in the 95,000 dalton region noted previously and several other smaller polypeptides of discreet size between 40,000 to 95,000 daltons. Thus the 120,000 protein is a polyhedrin fusion product and the smaller polypeptides represent alternate forms of the fusion product. The specific activity of beta-galactosidase in L1GP-gal3-infected cells is exceptionally high. The highest specific activity reported in yeast is one-half that observed in L1GP-gal3-infected cells at 48 hours post infection. The 33,000 dalton protein found in wild type infected cells is replaced in L1GP-gal3-infected cells by a polyhedrin/beta-galactosidase fusion protein of approximately 120,000 daltons as well as a series of other proteins of smaller molecular weight. The smaller proteins are individually less abundant than the 120,000 dalton protein and may arise by proteolytic cleavage of the 120,000 dalton fusion protein.

## Example 2

## Expression of Polyhedrin/Beta-galactosidase

## in other Cell Lines

The polyhedrin/beta-galactosidase fusion protein is also expressed by other insect cells which are permissive of AcNPV replication. For example, a cell line derived from the lepidopteran noctuid Trichoplusia ni, TN-368, was propagated in TC-100 media (Microbiological Associates) supplemented with 0.26% tryptose broth, 10% fetal bovine serum, 2mM L-glutamine and an antibiotic-antimycotic preparation (Gibco). Monolayers of this cell line were infected with various dilutions of the recombinant virus

L1GP-gal3 and overlaid with agarose-media containing 120 micrograms per milliliter of the B-galactosidase color indicator, X-gal. The monolayers were incubated at 27°C for 4 days and then color was developed by warming the plates to 37°C for 4-6 hours. The expression of the polyhedrin/B-galactosidase fusion protein was demonstrated by the vivid blue plaques on the T.ni monolayers. The titers indicated that the recombinant virus was approximately equally infectious on the T.ni monolayers as on S. frugiperda monolayers.


## Example 3


### A Generalized Transplacement Vector


For some types of genetic engineering applications it will be preferred to insert passenger genes under their own promoter control into the vector. In one such application, the transplacement vector plasmid pGP-B6874/Sal was constructed. This plasmid has been inserted in E.coli HB 101, a sample of which has been deposited at the American Type Culture Collection, and is held under ATCC accession number _____ . This vector provides a single Pst I site for the insertion of a passenger DNA under the control of its own or other exogenous promoter and allows the recombinant plasmid to be inserted into AcNPV using "blue" plaques as the means for selecting recombinant viruses.

The pGP-B6874/Sal plasmid illustrated in Fig. 4, was constructed by partially digesting pGP-B6874 with the restriction endonuclease Sal I and religating at low DNA concentration. Following transformation of E. coli HB 101, blue colonies were selected on kanamycin plates and then tested for sensitivity to ampicillin. The plasmids of

Amp$^S$, Kan$^R$, blue colonies were characterized by restriction endonuclease analysis. The pGP-B6874/Sal plasmid was shown to contain the regions beta-gal including the lac Z (beta-galactosidase gene, "Z") lac Y, ("Y"), Rep and Kan$^R$ genes to the Sal I site of Eco RI-I (S). The pGP-B6874/Sal plasmid is essentially pMC874 bordered by the N terminus and C terminus of the polyhedrin gene as in pGP-B6874. The Pst I site is not essential for beta-galactosidase expression or replication in E. coli. E. coli containing pGP-B6874/Sal are blue on x-gal plates and red on Maconkey plates.

Thus a transplacement plasmid, pGP-B6874/Sal has been constructed and may be used to facilitate the insertion and selection of recombinant viruses carrying passenger genes which are under the control of their own promoters. Passenger genes can be inserted at the unique Pst I site next to the E.coli replication genes of the plasmid and cloned in E. coli. The co-transfection of the resulting plasmid with wild type AcNPV and subsequent selection for blue viruses, should facilitate the selection of recombinant viruses.

To demonstrate the utility of pGP-B6874/SAL as a transplacement vector and to demonstrate the effective use of an exogenous promoter, the E. coli gene encoding chloramphenicol acetyl transferase (CAT) attached to the exogenous promoter, RSV-LTR, was inserted into pGP-B6874/SAL. The scheme for this construction is found in Figure 7. The plasmid pRSV-CAT (Gorman et al, Proc. Natl Acad Sci. 79: 6777 (1982)) was digested with PstI and BamHI. The transplacement plasmid pGP-B6874/SAL was digested with PstI and partially digested with BamHI. The two restriction endonuclease-digested plasmids were mixed and ligated. The resulting recombinant plasmids were introduced into E. coli by transformation and lac$^+$ colonies were selected on minimal media. The plasmid pLC-1 (see Fig. 7) was isolated and its structure verified by restriction endonuclease analysis.

S. frugiperda cell monolayers were transfected with a mixture of AcNPV L-1 DNA and pLC-I DNA in a ratio of 1 to 100. Viruses producing plaques with blue color and lacking OV were selected. A virus, AcNPV LI-RSV-CAT, was isolated and shown to contain the pLC-1 genes lac Z, lac Y, at least part of A, ("A"), ori(origin of replication, sometimes referred to as "rep") and RSV-LTR-CAT by restriction endonuclease analysis.

The AcNPV-Ll-RSV-CAT virus expressed the CAT gene as early as 2 hours post-infection as shown by assaying the CAT activity by the procedure of Gorman et al supra. Activity was first observed at 2 hours post-infection and continued to increase through 6 hours and 12 hours post infection. Thus an exogenous promoter, the RSV-LTR can be used to express exogenous genes in insect cells. Futhermore, an exogenous promoter can be used to express exogenous genes early in infection.

Many features of AcNPV make this virus a good host/vector for the propagation and expression of passenger genes in a metazoan environment. The rod-shaped capsid extends to accomodate additional DNA sequences and vector capacity may exceed 100 kilobases. Since extracellular virus is the infectious form in cell culture, the genes for occlusion are non-essential. The genes encoding polyhedrin or the 7.2K protein are ideal sites for passenger gene insertion since they are abundantly expressed between 18 and 72 hours post infection. At late times in

0155476

infection these proteins may constitute 20% or more of
the total protein of the infected cell. The locations of
the polyhedrin and 7.2K protein genes on the AcNPV physi-
cal map and the directions of the transcription are
known. The synthesis of the 1.2 kilobase polyhedrin
messenger RNA is temporally controlled and is first
detected at twelve hours post infection. The late expression (18 hours post infection) from the polyhedrin
promoter is particularly advantageous in expressing cytotoxic gene products.

The above examples demonstrate that AcNPV can be successfully genetically engineered and employed as a recombinant DNA vector. The examples also demonstrate that the
polyhedrin promoter and other promoters can drive the
high level expression of a passenger gene such as E. coli
beta-galactosidase. The specific activities of beta-
galactosidase achieved are significantly higher than
any previously reported in other prokaryotic or eukaryotic expression systems to the best of the applicant's
knowledge.

Additional advantages and modifications will readily occur
to those skilled in the art from a consideration of this
disclosure or practice of this invention, and it is
intended that this invention be limited only by the scope
of the appended claims.

What is claimed is:

1. A vector for production of a gene product by insect cells, comprising an exogenous gene segment which encodes the expression of the gene product, the gene segment being under the control of a promoter which promotes the expression of a gene product by insect cells.

2. The vector of claim 1, wherein the vector comprises a baculovirus.

3. The vector of claim 2, wherein the baculovirus comprises a nuclear polyhedrosis virus.

4. The vector of claim 3, wherein the nuclear polyhedrosis virus comprises an *Autographa californica* nuclear polyhedrosis virus or variant thereof.

5. The vector of claim 1, wherein the promoter is a viral promoter which is active in the late stages of viral growth to produce a viral gene product.

6. A vector of claim 5, wherein the viral promoter comprises a polyhedrin promoter.

7. The vector of claim 5, wherein the viral promoter

comprises a 7.2K promoter.

8. The vector of claim 1, wherein the promoter comprises a promoter which is active in the early stages of viral growth to produce a viral gene product.

9. The vector of claim 1, wherein the promoter comprises an exogenous promoter which is active in insect cells.

10. The vector of claim 1, wherein the exogenous segment comprises a gene segment which encodes the expression of a readily detectable gene product.

11. The vector of claim 10, wherein the readily detectable gene product comprises a protein which governs resistance to an antibiotic or a protein which reacts with an indicator to produce a detectable physical change.

12. The vector of claim 1, wherein the vector comprises the plasmid pGP-B6874.

13. The vector of claim 1, wherein the vector comprises the plasmid pGP-B6874/SAL.

14. The vector of claim 1, wherein the vector comprises the plasmid pLC-1

15. A method of preparing an expression vector suitable for use in insect cells, comprising inserting an exogenous gene segment into a DNA segment to form a vector, the insertion being made in a region of the DNA segment which is controlled by a promoter which promotes expression of a gene product by insect cells.

16. The method of claim 15, wherein the vector comprises a nuclear polyhedrosis virus.

17. The method of claim 15, wherein the DNA segment is derived from a virus which replicates in insect cells, the DNA segment comprises a gene region and a promoter region which controls the expression of the gene product encoded for in the gene region, and the insertion is made by providing a plasmid containing the DNA segment, and inserting the exogenous gene segment into the gene region of the DNA segment to form a recombinant plasmid vector.

18. The method of claim 17, wherein a viral vector is formed by contacting the recombinant plasmid vector with a virus which replicates in insect cells, and selecting for resultant viruses which express a gene product coded for by exogenous gene segment.

19. The method of claim 18, wherein the virus' is a nuclear polyhedrosis virus, the DNA segment comprises the polyhedrin promoter and at least a part of the polyhedrin gene, and the insertion occurs at a site within the polyhedrin gene region.

20. The method of claim 19, wherein the nuclear polyhedrosis virus comprises an _Autographica californica_ nuclear polyhedrosis virus or variant thereof.

21. The method of claim 15, wherein the promoter comprises a viral promoter.

22. The method of claim 21, wherein the viral promoter is a promoter which is active in the late stages of viral growth to produce a viral gene product.

23. The method of claim 15, wherein the promoter is a viral promoter which is active in the early stages of viral growth.

24. The method of claim 15, wherein the promoter is an exogenous promoter which is active in insect cells.

25. The method of making a gene product comprising inserting a vector into insect cells, said vector comprising an exogenous gene segment which codes for expression of the gene product, said exogenous gene segment being under the control of a promoter which promotes expression of a gene product in insect cells, culturing the insect cells under conditions favoring expression of the gene product, and recovering the gene product expressed.

26. The method of claim 25, wherein the vector comprises a nuclear polyhedrosis virus.

27. The method of claim 26, wherein the nuclear polyhedrosis virus comprises an <u>Autographa california</u> nuclear polyhedrosis virus or variant thereof.

28. The method of claim 25, wherein the promoter comprises a viral promoter.

29. The method of claim 28, wherein the viral promoter is a promoter which is active in the late stages of viral growth to produce a viral gene product.

30. The method of claim 29, wherein the viral promoter is a polyhedrin promoter.

31. The method of claim 29, wherein the promoter is a 7.2K promoter.

32. The method of claim 25, wherein the promoter is a viral promoter which is active in the early stages of viral growth to produce a viral gene product.

33. The method of claim 25, wherein the promoter is an exogenous promoter which is active in insect cells.

34. A gene product made in accordance with claim 25.

FIG.1

PHYSICAL MAP AcMNPV-LI DNA

FIG.2

% Genome

FIG. 3

FIG.6

FIG.4

FIG.5

FIG.7

European Patent Office

**EUROPEAN SEARCH REPORT**

01 5547 6

Application number

| | DOCUMENTS CONSIDERED TO BE RELEVANT | | EP 85101009.0 |
|---|---|---|---|

| Category | Citation of document with indication, where appropriate, of relevant passages | Relevant to claim | CLASSIFICATION OF THE APPLICATION (Int Cl 4) |
|---|---|---|---|
| D,A | SCIENCE, vol. 219, no. 4585, February 11, 1983 (Washington DC.) L.K. MILLER et al. "Bacterial, Viral, and Fungal Insecticides" pages 715-721 * Pages 718, 719 * -- | 1-4 | C 12 N 15/00 C 12 N 7/00 C 12 N 5/00 //C 12 R 1:91 |
| D,A | JOURNAL OF VIROLOGY, vol. 44, no. 3, December 1982 (Washington DC.) M.J. ADANG et al. "Molecular Cloning of DNA Complementary to mRNA of the Baculovirus Autographa californica Nuclear Polyhedrosis Virus: Location and Gene Products of RNA Transcripts Found Late in Infection" pages 782-793 * Abstract (page 782) * -- | 1-4 | |
| D,A | JOURNAL OF VIROLOGY, vol. 39, no. 3, September 1981 (Washington DC.) L.K. MILLER: "Construction of a Genetic Map of the Baculovirus Autographa californica Nuclear Polyhedrosis Virus by Marker Rescue of Temperature-Sensitive Mutants" pages 973-976 * Totality * ---- | 1-4 | TECHNICAL FIELDS SEARCHED (Int Cl 4) C 12 N |

The present search report has been drawn up for all claims

| Place of search | Date of completion of the search | Examiner |
|---|---|---|
| VIENNA | 03-05-1985 | WOLF |